# EUROPEAN PATENT SPECIFICATION

(11) **EP 0 341 870 B1**
(45) Date of publication and mention of the grant of the patent: **15.06.1994**
(21) Application number: 89304288.7
(22) Date of filing: 28.04.1989
(51) Int. Cl.: A61L 15/16, D04H 1/56

(54) **Absorbent elastomeric wound dressing**
Absorbierender elastomerischer Wundverband
Pansement absorbant élastomère pour blessure

(30) Priority: 13.05.1988 US 194082
(43) Date of publication of application: 15.11.1989
(73) Proprietor: MINNESOTA MINING AND MANUFACTURING COMPANY, St. Paul, Minnesota 55133-3427 (US)
(72) Inventor: Riedel, John E., St. Paul Minnesota 55144-1000 (US)
(74) Representative: Baillie, Iain Cameron

(56) References cited:
- EP-A- 0 174 775
- EP-A- 0 212 284
- EP-A- 0 333 209
- US-A- 4 707 398
- US-A- 4 724 184
- US-A- 4 741 949

## Description

### Background of the Invention

### Field of the Invention

The present invention relates to absorbent elastomeric wound dressings which provide rapid absorption, good wicking, and high liquid retention.

### Background Information

A wound dressing having high sorbency and retention, high conformability, low density, and good wicking, and which is non-linting and economical has long been desired. Gauzes are today the most common wound dressing, but gauzes are expensive to make and exhibit some linting which is undesirable. Many attempts have been made to find a substitute for gauzes, but until now, no fully satisfactory substitute has been found.

One effort in absorbent products has involved incorporating absorbent additives in a fibrous web. Dressings capable of rapid uptake of wound exudate and subsequent retention of those fluids typically rely on absorptive media having low solidity or high void volume. Unfortunately, high void volume webs typically have low strength and hence poor integrity, a combination which generally results in the dressing tearing apart and leaving residues in the wound site when the dressing is removed. Additionally, when these dressings tear or are compressed during removal, they release fluids which have been absorbed, thereby soiling or contaminating patient bedding or clothing.

U.S. Patent No. 3,670,731 (Harmon) discloses the addition of hydrocolloidal particles to a fibrous mass, such as wood pulp, by cascading the particles into the fibrous mass. However, mechanical action causes the particles to dust out.

U.S. Patent No. 4,366,814 (Riedel) discloses an elastic bandage material for medical tapes and dressing which has at least 50 percent by weight of an extensible porous fabric capable of elongation of at least 30 percent in one direction without tearing and at least 15 percent by weight of an elastomer uniformly impregnated in the fabric and substantially contained on or within the fibers of the fabric without filling the spaces between fibers. The fabric may be of a wide range of synthetic or natural fibers, used singly or in blends. The preferred elastomers include block copolymers, polyurethanes, acrylics, acrylic-olefinic copolymers, and other natural and synthetic rubbers.

U.S. Patent No. 4,414,970 (Berry) discloses a moisture vapor transmitting elastic bandage which has an inner layer of fabric and an outer layer of fabric bonded to a central layer which is an elastomeric film. The film can be continuous, macroporous or microporous, but is preferably continuous to provide a bacterial barrier. Suitable films which may be obtained in continuous form and which transmit moisture vapor can be made from polyurethane, for example, a thermoplastic polyurethane.

U.S. Patent No. 4,565,736 (Stein et al.) discloses a surgical compress which is made of an absorptive layer and a wound covering layer, the covering layer spun or otherwise made of nonwoven hydrophobic, hydrolysis-resistant, aliphatic polyurethane fibers, the covering layer preferably being autogenously bonded to the absorption layer by direct formation of tacky cover layer fibers on the absorption layer.

U.S. Patent No. 4,715,857 (Juhasz et al.) disclose wound dressings which comprise, in order, a first layer of a permeable material, a layer of a semi-permeable, adhesive material, a charcoal cloth or felt, and a second layer of a permeable material, in which the three layers a substantially co-extensive and surround the charcoal cloth or felt, whereby the first layer of permeable material is bound to the cloth or felt and, around the cloth or felt, to the second layer of permeable material. The layers of permeable material are in the form of a fabric or film and may be of different or, preferably, the same material, examples of suitable materials being natural or synthetic rubber, nylon, polyester, polyurethane and rayon acetate, and other suitable synthetic polymers. The semi-permeable adhesive materials are preferably double-sided transfer tapes.

British Patent Specification 1,575,830 (Johnson & Johnson) discloses a flexible and conformable disposable absorbent dressing which comprises a layer of absorbent material, and a thin, flexible, elastic and easily stretchable thermoplastic backing film retained in superimposed relationship with the absorbent layer, the backing film possessing an elastic recovery from 50 percent stretch of at least 75 percent, a rubber modulus of not above 2000 pounds per square inch and a Gurley stiffness at a thickness of 1 mil of not above one. The film is preferably formed from A-B-A block copolymers which consist of A end blocks derived from styrene and B blocks derived from conjugated dienes.

U.S. Patent No. 4,650,479 (Insley) discloses a sorbent sheet product comprising a coherent fibrous web that includes entangled blown polymeric fibers and high sorbency, liquid sorbent fibers intermingled with the blown polymeric fibers. The blown polymeric fibers may be formed from a wide variety of fiber-forming materials. Representative polymers for forming melt-blown fibers include polypropylene, polyethylene, polyethylene terephthalate, and polyamides.

European Patent Publication No. 0,156,649 (Insley et al.) discloses sorbent sheet products comprising a coherent fibrous web that includes entangled blown fibers and liquid transport fibers intermingled with the blown fibers and an array of solid high sorbency liquid-sorbent polymeric particles uniformly dispersed and physically held within the web. The particles swell upon sorption of liquid, and the transport fibers cause increased and more rapid sorption of liquid by conducting the liquid from external portions of the web to internal portions of the web.

U.S. Patent No. 4,692,371 (Morman et al.) discloses elastomeric nonwoven webs of elastomeric meltblown fibers, elastomeric films or elastomeric molded materials attained by forming styrenic-(ethylenebutylene)-styrenic block copolymers at elevated temperatures of at least about 290°C. In the case of elastomeric nonwoven webs of elastomeric fibers, other fibers, such as pulp or cellulosic fibers or nonfibrous material such as particulates, may be combined with the elastomeric fibers by known methods.

U.S. Patent No. 4,692,368 (Taylor et al.) discloses a laminate which is elastic in at least one direction and includes an elastic sheet having at least one nonelastic, nonwoven web joined thereto at least at two areas. The nonelastic web is gathered between the two areas. The elastic sheet is formed from an aromatic polyetherurethane, preferably in the form of melt blown fibers. The nonelastic nonwoven web includes spunlaced hydraulically entangled polyester fibers. The nonelastic nonwoven web may also include rayon or wood pulp fibers.

U.S. Patent No. 4,118,531 (Hauser) discloses a web of blended microfibers and crimped bulking fibers. The web is a lofty resilient web which has high thermal resistance per unit of thickness and moderate weight, as well as other properties which give the web a distinctive utility as thermal insulation.

EP-A-0 333 209 which is available as prior art only for the purposes of Articles 54(3) and 54(4) EPC, discloses nonwoven fibrous elastomeric web material which is a hydraulically entangled conform or admixture of (1) melt-blown fibers such as elastic melt-blown fibers and (2) pulp fibers and/or staple fibers and/or melt-blown fibers and/or continuous filaments, with or without particulate material. EP-A-0 333 209 fails to teach or suggest the amounts of various fibers present and, in fact, in the examples shows only webs having two components. EP-A-0 333 209 does not teach or suggest the balanced component amounts required in the wound dressing material of the present invention.

US-A-4,741,949, US-A-4,724,184 and US-A-4,707,398 disclose elastomeric nonwoven webs which may also include nonelastic fibers and/or particulate materials. At most these references contemplate three component mixtures and again fail to teach or suggest the balanced component amounts required in the wound dressing material of the present invention.

EP-A-0 174 775 discloses a sorbent sheet product of entangled blown polymeric fibers, high sorbency, liquid sorbent fibers and, optionally liquid transport fibers. Again this reference contemplates at most three component mixtures and does not suggest the use of elastomeric melt-blown fibers.

EP-A-0 212 284 discloses a gathered, nonwoven elastic web which is a composite if a nonwoven elastic web and a nonwoven gatherable web. Even combining the teachings of the references would not lead one to the four component absorbant web of the present invention which has an excellent balance of properties for wound dressing.

### Summary of the Invention

This invention provides an elastomeric nonwoven absorbent web, particularly useful in wound dressings, comprising a nonwoven fibrous matrix of (1) 10 to 20 weight percent elastomeric melt-blown 5-30 »m small diameter fibers, (2) 10 to 30 weight percent absorbent staple fibers or 10 to 20 weight percent absorbent polymeric particulate material, (3) 30 to 60 weight percent wicking staple fibers, and (4) 10 to 30 weight percent crimped bulking staple fibers dispersed throughout the matrix.

This invention further provides an absorbent elastomeric wound dressing comprising (a) a fluid permeable, compliant, low adherency wound contacting layer, (b) an intermediate conformable, fluid-absorbent element, the element having a nonwoven fibrous matrix of (1) 10 to 20 weight percent elastomeric melt-blown 5-30 »m small diameter fibers (2) 10 to 30 weight percent absorbent staple fibers or 10 to 20 weight percent absorbent polymeric particulate material, (3) 30 to 60 weight percent wicking staple fibers, and (4) 10 to 30 weight percent crimped bulking staple fibers dispersed throughout the matrix, and (c) a soft, compliant cover layer.

The elastomeric webs have excellent wound exudate management properties, good integrity, strength, and the ability to accept extremely high, e.g., 85 weight percent or more, loading levels of secondary fibers and particulate materials. Furthermore, extension or flexing of the webs does not cause the secondary fibers or particulate materials to become dislodged or dust out. Also, the loaded webs retain their conformability even under high loadings.

In addition to having excellent wound exudate management properties, the dressings of the present invention are significantly more elastic and conformable than conventional dressings. This conformability coupled with the high void volume of the dressing allows the dressings to be used as a packing material for cavernous wounds. The integrity of the dressings is such that even when they are used as packings, they are readily removed without tearing and releasing exudates.

### Detailed Description of the Invention

The wound dressings of the present invention are based on absorbent materials which utilize elastomeric nonwoven webs as a delivery matrix for a variety of vehicles useful for wound management. Exemplary elastomeric materials which can be used to prepare the nonwoven elastomeric webs include polyurethane elastomeric materials, polyester elastomeric materials, polyamide elastomeric materials, and A-B-A block copolymer materials where the A end groups are styrenic moieties and B is an elastomeric midblock. Particularly preferred are polyurethane elastomeric materials. The nonwoven elastomeric webs are preferably formed as a melt-blown web of small diameter fibers as described, for example, in Wente, Van A. "Superfine Thermoplastic Fibers," in Industrial Engineering Chemistry, Vol. 48, pages 1342 et seq (1956). The elastomeric meltblown small diameter fibers have an average diameter of 5 to 30 microns. The elastomeric melt-blown small diameter fibers are present in the elastomeric nonwoven absorbent web in an amount of 10 to 20 weight percent, preferably 12 to 18 weight percent.

Retention of exudate fluids is accomplished by loading water-insoluble, water-absorbing, super absorbent particles or absorbent staple fibers which rapidly absorb and retain under pressure large quantities of liquids into the web. The preferred super absorbent particles include modified starches, examples of which are described in U.S. Patent No. 3,981,100, and high molecular weight acrylic polymers containing hydrophilic groups. A wide variety of such water-insoluble, water-absorbing particles are commercially available and they typically absorb 20 or more times their weight of water, preferably 100 or more times their weight of water. In general, the absorbent particles should absorb at least their own weight of liquid. The sorbent particles may vary in size, at least from 50 to 3000 micrometers in average diameter. Preferably, the particles are between 75 and 1500 micrometers in average diameter.

When the absorbent material is super absorbent particulate material,the particulate is present in the delivery matrix in amounts of 10 to 20 weight percent of the total weight of the absorbent nonwoven web.

Fibers useful as absorbent staple fibers in the present invention are those having an absorbency of at least 1000% when tested according to ASTM Test Method D-117. To achieve high liquid absorbency and good liquid retention under pressure, the absorbent staple fiber should have at least one outside portion of highly hydrophilic material. Examples of such highly hydrophilic fibers are those prepared by treating acrylonitrile fibers with an alkali metal hydroxide to form a hydrophilic crosslinked polymer on the surface thereof as disclosed in U.S. Patents No. 4,366,206 and No. 4,374,175. Also useful are fibers having a sorbent coating such as a crosslinked, saponified copolymer of methacrylic acid and ethacrylic acid or a homopolymer of acrylic acid. A particularly useful fiber is Lanseal™ F, an acrylonitrile fiber having a hydrophilic crosslinked polymer on the surface thereof, available from Japan Exlan Co., Ltd., Osaka, Japan.

The size of the absorbent staple fibers is preferably in the range of 0.5 to 50 denier, more preferably 1 to 30 denier. The size of the sorbent staple fibers depends on the end use of the product with absorbent staple fibers of lower denier providing a softer hand. Preferably, the fibers have an average length in the range of 2 to 15 centimeters, more preferably less than 7 to 10 centimeters. The absorbent staple fibers may be crimped to provide additional freedom of expansion to the product during liquid absorption as well as bulk and resilience.

When the absorbent material used in the nonwoven absorbent web is absorbent staple fiber, the fiber is present in an amount of 10 to 30 weight percent of the total nonwoven absorbent web, preferably 17 to 27 weight percent of the total nonwoven absorbent web. Of course, a combination of super absorbent particles and absorbent fibers can be used to provide absorptive capacity in the nonwoven absorbent web.

Wound exudate management properties can be enhanced by further loading the nonwoven absorbent web with hydrophilic wicking fiber to facilitate movement of the exudate fluids from the wound surface into the central absorbing element of the dressing. Fibers useful as wicking fibers are staple fibers having a water retention value of at least 10%, preferably 20%, and more preferably 25% when tested according to ASTM Test Method D2402. Fibers which can be used as wicking fibers include, for example, rayon or cotton staple fibers. Particularly preferred wicking fibers are Absorbit™ rayon staple fiber, available from American Enka Company and Avtex™ Regular rayon staple fiber, available from Avtex Corp. Fiber loading levels of from 30 to 60 weight percent of the web are used to achieve the desired wicking performance. Preferably, loading levels of the wicking fibers range from 10 to 35 weight percent of the nonwoven absorbent structure.

In addition to the super absorbent particles or fibers and the wicking fibers, it is also necessary to incorporate crimped bulking fibers into the absorbent web matrix. The bulking fibers are staple fibers which assist in retaining the open structure of the nonwoven web to facilitate transfer of wound exudate into the matrix and to prevent the absorbed fluids from being forced out of the absorbent web when compressive force is applied to the absorbent nonwoven web. The bulking fibers should, as a minimum, have an average length sufficient to include at least one complete crimp and preferably at least three or four crimps. The bulking fibers preferably have an average length between 2 and 15 cm, more preferably the bulking fibers are less than 7-10 cm in length. Preferably, the bulking fibers are moderately stiff, that is, have a flexural rigidity of 1.5 x 10⁻⁴ gram-square centimeters per tex or more (as defined by W.E. Morton and J.W.S. Hearle, Physical Properties of Textile Fibers, Butterworth, London, 1962, pp. 380-383).

Useful crimped bulking fibers include, for example, acrylic, polyester, nylon, polyolefin, rayon and acetate fibers. Polyester and acrylic fibers are particularly preferred. The crimped bulking fibers are incorporated at loading levels of from 10 to 30 weight percent of the absorbent nonwoven web.

In addition to the staple fibers, i.e., the absorbent staple fibers, the wicking staple fibers and the bulking staple fibers, and particulate materials used for exudate management considerations, other fibers or particulate matter may be incorporated into the absorbing matrix for the purpose of controlling odors and providing anti-bacterial activity to the dressing. Such other fibers or particulate matter preferably comprise less than 30 weight percent of the elastomeric nonwoven absorbent web.

The various particulate materials and staple fibers to be incorporated into the elastomeric nonwoven absorbent web can be incorporated by well known methods such as are described in U.S. Patent No. 4,755,178, (Insley).

Preferably, the nonwoven absorbent webs of the invention have a softness value of less than 80 g, more preferably less than 60 g, most preferably less than 50 g, when tested according to INDA Test Procedure 90.0-75, using a slot width of 10 mm. Preferably, the nonwoven absorbent webs have a wicking rate of at least 0.5 cm, more preferably at least 1.5 cm, most preferably at least 2 cm, when tested according to INDA Test Procedure 10.3-70. Preferably, the nonwoven absorbent webs have an absorbency of at least 1000%, more preferably at least 1200%, most preferably at least 1300%, when tested according to ASTM Test Method D 117 or D-461 using a 1% saline solution. Preferably, the nonwoven absorbent webs of the invention have a fluid retention value of at least 50%, more preferably at least 55%, most preferably at least 60%, when tested according to ASTM Test Method D 461 using a 1% saline solution and a 475 g roller.

A variety of materials can be used for the fluid permeable, compliant, low adherency wound contacting layer of the absorbent dressings of the present invention. This wound contacting layer should be sufficiently permeable to permit good flow of wound exudate through the layer and into the nonwoven absorbent layer. Fluid flow must be sufficient to prevent fouling of the exudate under the dressing wound contact layer. Materials useful for the wound contacting layer include, for example, small fiber diameter melt-blown nonwoven webs, porous polyethylene films, woven nylon fabrics, perforated polyethylene film and blown microfiber polypropylene webs. The wound contacting layer of the absorbent wound dressing is preferably coextensive with the nonwoven absorbent web.

Materials suitable for use as the a soft, compliant cover layer or non-wound contacting layer of the absorbent dressings of the present invention preferably have high moisture vapor permeability and low liquid permeability. Such materials include, for example, high moisture vapor permeable films, porous foam material and nonwoven webs. The cover layer can be of the same material as the wound contacting material or other materials such as pressure-sensitive adhesive coated materials. The cover layer may be coextensive with the wound contacting and nonwoven absorbent web or, when the cover layer is coated with a pressure-sensitive adhesive material, the cover layer may extend beyond the dimensions of the wound contacting and absorbent layers to provide a means for securing the dressing over the wound site. In the event that the cover layer does not extend beyond the wound contacting and absorbent layers, the dressing can be secured over the wound with a cohesive wrap or tape.

This invention is further illustrated by the following examples, but the particular materials and amounts thereof recited in these examples, as well as other conditions and details, should not be construed to unduly limit this invention.

In the examples, all parts and percentages are by weight unless otherwise specified. The following test methods were used for evaluation purposes in the examples:
Softness value: INDA Test Procedure 90.0-75, using a slot width of 10 mm;
Wicking rate: INDA Test Procedure 10.3-70.
Absorbency: ASTM Test Method D 117 or D-461 using a 1% saline solution; and
Fluid retention value: ASTM Test Method D 461 using a 1% saline solution and a 475 g roller.

### Examples 1-19

In Example 1, an elastomeric, nonwoven, absorbent, melt-blown, microfiber web was prepared using thermoplastic elastomeric polyurethane polymer (PS 455-203, a polyesterurethane available from K.J. Quinn Co., Malden, MA) as the delivery matrix, and a fiber blend of 15 weight percent absorbent fiber (Lanseal™ F, 2.5 denier, 51 mm staple length, available from Japan Exlan Co., Ltd., Osaka, Japan), 70 weight percent wicking fiber (Avtex™ Regular, a rayon fiber, 15 denier, 40 mm staple length, available from Avtex Corp., and 15 weight percent bulking fiber (Orlon™ Type OT 670, 3 denier, 38 mm staple length, available from DuPont Co.).

The webs were prepared using a melt blowing process similar to that taught in U.S. Patent No. 4,188,531 (Hauser) except that the melt-blowing die had circular smooth surfaced orifices (10/cm) with a 5:1 length-to-diameter ratio. The die temperature was maintained at 220°C, the primary air temperature and pressure were, respectively, 230°C and about 150 kPa, (0.63 mm gap width), and the polymer throughput rate was 450 gm/hr/cm. The resulting web had a basis weight of 120 g/m² and contained 18.3 weight percent polyurethane microfiber having a fiber size of 5 to 10 microns and 81.7 weight percent staple fibers.

In Examples 2-19, elastomeric nonwoven absorbent webs were prepared as in Example 1, except that the staple fiber content and basis weight were varied as set forth in Table I. In addition to the Lanseal™ F, rayon, and Orlon™ (identified in Table I as L-F, R, and O, respectively), SA 700, an absorbent web available from Arco, Philadelphia, PA and Lanseal™ FA, 3 denier, 51 mm staple length, an odor and fluid absorbent fiber available from Japan Exlan Co., Ltd., Osaka, Japan (identified in Table I as Arco and L-FA, respectively) were used in these examples. The total basis weight of the elastomeric nonwoven absorbent webs are also set forth in Table I.

**Table I**

| Example | Microfiber (wt %) | Staple Fiber Blend Content (weight percent) | | | | | Basis weight (g/m²) |
|---|---|---|---|---|---|---|---|
| | | L-F | Arco | L-FA | R | O | |
| 1 | 18.3 | 15 | - | - | 70 | 15 | 120 |
| 2 | 17.6 | 15 | - | - | 60 | 25 | 125 |
| 3 | 19.3 | - | 15 | - | 60 | 25 | 114 |
| 4 | 16.9 | 20 | - | - | 60 | 20 | 130 |
| 5 | 12.9 | 20 | - | - | 60 | 20 | 170 |
| 6 | 16.7 | - | 20 | - | 60 | 20 | 132 |
| 7 | 13.2 | - | 20 | - | 60 | 20 | 167 |
| 8 | 16.7 | - | 30 | 10 | 40 | 20 | 135 |
| 9 | 13.7 | - | 30 | 10 | 40 | 20 | 160 |
| 10 | 17.5 | 30 | - | 20 | 35 | 15 | 126 |
| 11 | 12.8 | 30 | - | 20 | 35 | 15 | 172 |
| 12 | 18.2 | 30 | - | 10 | 45 | 15 | 121 |
| 13 | 12.4 | 30 | - | 10 | 45 | 15 | 178 |
| 14 | 17.0 | 20 | - | 10 | 55 | 15 | 129 |
| 15 | 13.6 | 20 | - | 10 | 55 | 15 | 162 |
| 16 | 16.9 | 30 | - | - | 50 | 20 | 130 |
| 17 | 13.5 | 30 | - | - | 50 | 20 | 163 |
| 18 | 16.5 | - | 30 | - | 50 | 20 | 133 |
| 19 | 12.7 | - | 30 | - | 50 | 20 | 173 |

The webs described in Table I were evaluated for softness, wicking, absorbency, absorbency as a function of basis weight and percent retention. Results of these evaluations are reported in Table II. Absorbency and fluid retention data were generated with a one percent saline solution. Unreported absorbency data generated with deionized water were approximately 1.5 times the values reported in Table II.

**Table II**

| Example | Softness (g) | Wicking (cm/30 sec) | Absorbency | | Retention (%) |
|---|---|---|---|---|---|
| | | | (%) | (g/g) | |
| 1 | -* | 0.9 | 1070 | 11.7 | - |
| 2 | - | 0.6 | 1220 | 13.3 | - |
| 3 | - | 1.6 | 1170 | 12.7 | - |
| 4 | 30 | 1.4 | 1280 | 13.8 | 60.6 |
| 5 | 55 | 1.8 | 1247 | 13.5 | - |
| 6 | 75 | 2.2 | 1425 | 15.3 | 60.1 |
| 7 | 110 | 2.0 | 1368 | 14.7 | - |
| 8 | 62 | 1.5 | 1470 | 15.8 | 61.2 |
| 9 | 94 | 1.7 | 1341 | 14.6 | - |
| 10 | 29 | 0.7 | 1342 | 14.5 | 60.5 |
| 11 | 39 | 1.0 | 1360 | 14.6 | - |
| 12 | 38 | 1.9 | 1320 | 14.2 | 60.7 |
| 13 | 42 | 1.6 | 1413 | 15.1 | - |
| 14 | 50 | 2.2 | 1247 | 13.5 | 60.9 |
| 15 | 54 | 1.9 | 1363 | 14.6 | - |
| 16 | 48 | 1.7 | 1230 | 13.3 | 60.1 |
| 17 | 54 | 1.5 | 1350 | 14.5 | - |
| 18 | 80 | 2.3 | 1447 | 15.5 | 62.2 |
| 19 | 122 | 1.9 | 1425 | 15.3 | - |

| | | | | | |
|---|---|---|---|---|---|
| * test not run | | | | | |

As can be seen from the data in Table II, absorbency wicking and softness are variables which are related to the fiber composition and the ratio of these fibers to each other. For example, the Arco fiber definitely makes a stiffer web.

### Examples 20-24

In Examples 20-24, elastomeric nonwoven absorbent webs were prepared as in Example 1, except that the elastomeric polyurethane polymer used in Examples 20-22 was PS 440-101, a polyesterurethane thermoplastic elastomeric resin available from K.J. Quinn Co., Malden, MA, the staple fiber blend contained 15 weight percent Lanseal™ F, 60 weight percent rayon, and 25 weight percent Orlon™, and the polyurethane fiber diameters, the percent of polyurethane microfiber in the web, and the basis weight of the total absorbent web were as set forth in Table III.

**Table III**

| Example | Microfiber diameter (») | Microfiber (wt %) | Basis weight (g/m₂) |
|---|---|---|---|
| 20 | 5 - 10 | 15.4 | 123 |
| 21 | 22 - 30 | 16.4 | 126 |
| 22 | 5 - 10 | 20.0 | 125 |
| 23 | 5 - 10 | 13.3 | 128 |
| 24 | 22 - 30 | 16.4 | 122 |

The webs described in Table III were evaluated for softness, wicking, absorbency, absorbency as a function of basis weight and percent retention. Results of these evaluations are reported in Table IV. Absorbency and fluid retention data were generated with a one percent saline solution. Unreported absorbency data generated with deionized water were approximately 1.5 times the values reported in Table IV.

**Table IV**

| Example | Softness (g) | Wicking (cm/30 sec) | Absorbency | | Retention (%) |
|---|---|---|---|---|---|
| | | | (%) | (g/g) | |
| 20 | 44 | 0.6 | 1210 | 13.1 | 61.2 |
| 21 | 52 | 0.7 | 1228 | 13.3 | 60.7 |
| 22 | 41 | 0.6 | 1235 | 13.3 | 63.0 |
| 23 | 48 | 0.6 | 1220 | 13.2 | 62.7 |
| 24 | 56 | 0.7 | 1220 | 13.2 | 61.4 |

As can be seen from the data in Table IV, there is little effect of fiber size on the two polyurethane resins on the critical properties for this dressing. There is an indication that increasing the polyurethane fiber content may actually provide a slightly softer material.

### Examples 25-34

In Examples 25-34, double thicknesses of the elastomeric nonwoven absorbent webs of Examples 10-17 and 24-25, respectively were tested for softness, wicking and absorbency. The results are set forth in Table V.

**Table V**

| Example | Web origin (Example) | Softness (g) | Wicking (cm/30 sec) | Absorbency | |
|---|---|---|---|---|---|
| | | | | (%) | (g/g) |
| 25 | 10 | 90 | 1.6 | 1438 | 15.6 |
| 26 | 11 | 175 | 2.0 | 1469 | 15.9 |
| 27 | 12 | 159 | 2.2 | 1423 | 15.4 |
| 28 | 13 | 371 | 2.3 | 1392 | 15.1 |
| 29 | 14 | 182 | 1.8 | 1496 | 16.2 |
| 30 | 15 | 299 | 1.9 | 1443 | 15.6 |
| 31 | 16 | 115 | 1.0 | 1396 | 15.1 |
| 32 | 17 | 172 | 1.4 | 1425 | 15.4 |
| 33 | 18 | 218 | 2.5 | -* | - |
| 34 | 19 | 311 | 2.0 | - | - |

| | | | | | |
|---|---|---|---|---|---|
| * test not run | | | | | |

As can be seen from the data in Table V, using two layers of absorbent web has no significant effect on any of the properties except softness which reflects the added thickness vs. the test method limitation.

### Comparative Examples 1-4

In comparative examples 1-4, commercially available wound dressings were tested for softness, wicking, absorbency, absorbency in relation to basis weight, and percent resiliency. The dressing of Comparative Example 1 was an 8-ply cotton gauze dressing, available from the Kendall Co., Boston, MA, which had a basis weight of 165 gm/m². The dressing of Comparative Example 2 was a Telfa™ dressing, available from the Kendall Co., Boston, MA, which had a basis weight of 197 gm/m². The dressing of Comparative Example 3 was a Melolin™ dressing, available from Smith & Nephew Medical, Ltd., England, which had a basis weight of 145 gm/m². The dressing of Comparative Example 4 was a Microdon™ dressing, available from 3M Company, St. Paul, MN, which had a basis weight of 241 gm/m². Absorbency data collected using deionized water showed essentially no difference from the data collected using one percent saline solution on these samples. The dressings were tested for softness, wicking, absorbency, absorbency in relation to basis weight, and percent resiliency. The results are set forth in Table VI.

**Table VI**

| Comparative Example | Softness (g) | Wicking (cm/30 sec) | Absorbency | | Retention (%) |
|---|---|---|---|---|---|
| | | | (%) | (g/g) | |
| 1 | 58 | 1.0 | 690 | 8.4 | 43 |
| 2 | 75 | 0.3 | 700 | 8.5 | 54 |
| 3 | 90 | 0.4 | 620 | 7.8 | 46 |
| 4 | 130 | 0.4 | 1020 | 11.1 | 56 |

As can be seen from the data in Table VI, absorbency retention and softness do not achieve the levels of the dressing examples of this application. Wicking rate on samples, 2, 3, and 4 is also significantly slower.

The various modifications and alterations of this invention will be apparent to those skilled in the art without departing from the scope and spirit of this invention and this invention should not be restricted to that set forth herein for illustrative purposes.

## Claims

1. An elastomeric nonwoven absorbent web comprising a nonwoven fibrous matrix of (1) 10 to 20 weight percent elastomeric melt-blown 5-30 »m diameter fibers, (2) 10 to 30 weight percent absorbent staple fibers or 10 to 20 weight percent absorbent polymeric particulate material, (3) 30 to 60 weight percent wicking staple fibers, and (4) 10 to 30 weight percent crimped bulking staple fibers dispersed throughout the matrix.

2. The elastomeric nonwoven absorbent web of claim 1 wherein said elastomeric melt-blown 5-30 »m diameter fibers comprise polyurethane elastomeric materials, polyester elastomeric materials, polyamide elastomeric materials, A-B-A block copolymer materials where the A end groups are styrenic moieties and B is an elastomeric midblock, and combinations thereof.

3. The elastomeric nonwoven absorbent web of claim 1 wherein said elastomeric melt-blown 5-30 »m diameter fibers comprise polyurethane fibers.

4. The elastomeric nonwoven absorbent web of any one of claims 1-3 wherein said absorbent staple fibers comprise fibers having an absorbency of at least 1000%.

5. The elastomeric nonwoven absorbent web of any preceding claim wherein said absorbent staple fibers comprise acrylonitrile fibers having a hydrophilic surface.

6. The elastomeric nonwoven absorbent web of any preceding claim wherein said wicking staple fibers comprise cotton fibers, rayon fibers, wool fibers, silk fibers or combinations thereof.

7. The elastomeric nonwoven absorbent web of any preceding claim wherein said bulking staple fibers are crimped fibers having an average crimp frequency of more than one-half crimp per centimeter.

8. The elastomeric nonwoven absorbent web of claim 7 wherein said crimp frequency is at least 2 crimps per centimeter.

9. The elastomeric nonwoven absorbent web of any preceding claim wherein said bulking staple fiber has a flexural rigidity of 1.5 x 10⁻⁴ grams-square centimeters per tex.

10. The elastomeric nonwoven absorbent web of any preceding claim wherein said bulking staple fiber is acrylic or polyester.

11. The elastomeric nonwoven absorbent web of any preceding claim wherein said web has a softness of less than 80 g.

12. The elastomeric nonwoven absorbent web of any preceding claim wherein said web has a wicking value of at least 0.5 cm/30 sec.

13. The elastomeric nonwoven absorbent web of any preceding claim wherein said web has an absorbency of at least 1000 percent.

14. The elastomeric nonwoven absorbent web of any preceding claim wherein said web has an absorbency of at least 11.5 g per g basis weight.

15. The elastomeric nonwoven absorbent web of any preceding claim wherein said web has a retention of absorbed liquid of at least 50 percent.

16. An absorbent elastomeric wound dressing comprising (1) a fluid permeable, compliant, low adherency wound contacting layer, (2) an intermediate conformable, fluid-absorbent element, the element comprising the elastomeric nonwoven absorbent web of any preceding claim and (3) a soft, compliant cover layer.

17. The wound dressing of claim 16 wherein said wound contacting layer is a porous nonwoven small diameter fiber web.

18. The wound dressing of any one of claims 16-17 wherein said cover layer is absorbent liquid permeable and moisture vapor permeable.

19. The wound dressing of claim 18 wherein one face of said cover layer comprises a pressure-sensitive adhesive layer for contacting with said fluid absorbent element.

## Patentansprüche

1. Elastomerer saugfähiger Vliesstoff mit einer Faservliesmatrix aus (1) 10 bis 20 Gew.-% schmelzgeblasenen elastomeren Fasern mit einem Durchmesser von 5 bis 30 Mikrometern, (2) 10 bis 30 Gew.-% saugfähigen Stapelfasern oder 10 bis 20 Gew.-% saugfähiger feinteiliger polymerer Substanz, (3) 30 bis 60 Gew.-% dochtartig wirksamen Stapelfasern und (4) 10 bis 30 Gew.-% gekräuselten Bauschfasern, die in der ganzen Matrix dispergiert sind.

2. Elastomerer saugfähiger Vliesstoff nach Anspruch 1, in dem die schmelzgeblasenen elastomeren Fasern mit einem Durchmesser von 5 bis 30 Mikrometern wenigstens teilweise aus elastomeren Polyurethansubstanzen, elastomeren Polyestersubstanzen, elastomeren Polyamidsubstanzen, A-B-A-Blockpolymersubstanzen, in denen die Endgruppen A Anteile auf Styrolbasis sind und B ein elastomerer Mittelblock ist, und Kombinationen derselben bestehen.

3. Elastomerer saugfähiger Vliesstoff nach Anspruch 1, in dem die schmelzgeblasenen elastomeren Fasern mit einem Durchmesser von 5 bis 30 Mikrometern wenigstens teilweise aus Polyurethanfasern bestehen.

4. Elastomerer saugfähiger Vliesstoff nach einem der Ansprüche 1 bis 3, in dem die saugfähigen Stapelfasern wenigstens teilweise aus Fasern mit einer Saugfähigkeit von mindestens 1000% bestehen.

5. Elastomerer saugfähiger Vliesstoff nach einem der vorhergehenden Ansprüche, in dem die saugfähigen Stapelfasern wenigstens teilweise aus Acrylnitrilfasern mit einer hydrophilen Oberfläche bestehen.

6. Elastomerer saugfähiger Vliesstoff nach einem der vorhergehenden Ansprüche, in dem die dochtartig wirksamen Stapelfasern wenigstens teilweise aus Baumwollfasern, Reyonfasern, Wollfasern, Seidenfasern oder Kombinationen derselben bestehen.

7. Elastomerer saugfähiger Vliesstoff nach einem der vorhergehenden Ansprüche, in dem die Bauschstapelfasern gekräuselte Fasern mit einer durchschnittlichen Kräuselfrequenz von mehr als einem halben Kräuselbogen pro cm sind.

8. Elastomerer saugfähiger Vliesstoff nach Anspruch 7, in dem die Kräuselfrequenz mindestens 2 Kräuselbogen pro cm beträgt.

9. Elastomerer saugfähiger Vliesstoff nach einem der vorhergehenden Ansprüche, in dem die Bauschstapelfasern eine Biegesteifheit von 1,5 . 10⁻⁴ g-cm² pro tex haben.

10. Elastomerer saugfähiger Vliesstoff nach einem der vorhergehenden Ansprüche, dadurch gekennzeichnet, daß die Bauschstapelfasern aus Acrylharz oder Polyester bestehen.

11. Elastomerer saugfähiger Vliesstoff nach einem der vorhergehenden Ansprüche, der eine Weichheit unter 80 g hat.

12. Elastomerer saugfähiger Vliesstoff nach einem der vorhergehenden Ansprüche, der eine Dochtwirkung von mindestens 0,5 cm/30 s hat.

13. Elastomerer saugfähiger Vliesstoff nach einem der vorhergehenden Ansprüche, der eine Saugfähigkeit von mindestens 1000% hat.

14. Elastomerer saugfähiger Vliesstoff nach einem der vorhergehenden Ansprüche, der eine Saugfähigkeit von mindestens 11,5 g pro g Rohgewicht hat.

15. Elastomerer saugfähiger Vliesstoff nach einem der vorhergehenden Ansprüche, der für aufgesaugte Flüssigkeit eine Retention von mindestens 50% hat.

16. Elastomerer saugfähiger Wundverbandstoff mit (1) einer fluiddurchlässigen, flexiblen, nur schwach Klebfähigen Schicht zum Auflegen auf die Wunde, (2) einer für Fluid saugfähigen Zwischenlage, die wenigstens teilweise aus dem elastomeren saugfähigen Vliesstoff nach einem der vorhergehenden Ansprüche besteht, und (3) einer weichen flexiblen Deckschicht.

17. Wundverbandstoff nach Anspruch 16, in dem die zum Auflegen auf die Wunde bestimmte Schicht ein poröses Faservlies aus Fasern von kleinem Durchmesser ist.

18. Wundverbandstoff nach einem der Ansprüche 16 bis 17, in dem die Deckschicht saugfähig, flüssigkeitsdurchlässig und wasserdampfdurchlässig ist.

19. Wundverbandstoff nach Anspruch 18, in dem eine Oberfläche der Deckschicht mit einer Haftkleberschicht versehen ist, die mit der für Fluid saugfähigen Zwischenlage in Berührung bringbar ist.

## Revendications

1. Feuille élastomère absorbante non tissée comprenant une matrice fibreuse non tissée de (1) 10 à 20% en poids de fibres élastomères soufflées à l'état fondu de 5 à 30 »m de diamètre, (2) 10 à 30% en poids de fibres discontinues absorbantes ou 10 à 20% en poids de polymère absorbant en particules, (3) 30 à 60% en poids de fibres discontinues de capillarité, et (4) 10 à 30% en poids de fibres discontinues frisées à effet de gonflant dispersées dans toute la matrice.

2. Feuille élastomère absorbante non tissée suivant la revendication 1, dans laquelle lesdites fibres élastomères soufflées à l'état fondu de 5 à 30 »m de diamètre comprennent des matières élastomères de type polyuréthane, des matières élastomères de type polyester, des matières élastomères de type polyamide, des copolymères séquencés A-B-A dans lesquels les groupes d'extrémité A sont des motifs styréniques et B est un bloc d'élastomère intermédiaire, et leurs combinaisons.

3. Feuille élastomère absorbante non tissée suivant la revendication 1, dans laquelle lesdites fibres élastomères soufflées à l'état fondu de 5 à 30 »m de diamètre comprennent des fibres de polyuréthane.

4. Feuille élastomère absorbante non tissée suivant une quelconque des revendications 1 à 3, dans laquelle lesdites fibres discontinues absorbantes comprennent des fibres ayant une absorbance d'au moins 1000%.

5. Feuille élastomère absorbante non tissée suivant une quelconque des revendications 1 à 3, dans laquelle lesdites fibres discontinues absorbantes comprennent des fibres d'acrylonitrile ayant une surface hydrophile.

6. Feuille élastomère absorbante non tissée suivant une quelconque des revendications précédentes, dans laquelle lesdites fibres discontinues de capillarité comprennent des fibres de coton, des fibres de rayonne, des fibres de laine, des fibres de soie ou leurs combinaisons.

7. Feuille élastomère absorbante non tissée suivant une quelconque des revendications précédentes, dans laquelle lesdites fibres discontinues de gonflant sont des fibres frisées ayant une fréquence moyenne de frisure de plus d'une demi-frisure par centimètre.

8. Feuille élastomère absorbante non tissée suivant la revendication 7, dans laquelle ladite fréquence de frisure est au moins de deux frisures par centimètre.

9. Feuille élastomère absorbante non tissée suivant une quelconque des revendications précédentes, dans laquelle ladite fibre discontinue de gonflant a une rigidité en flexion de 1,5 x 10⁻⁶ N/cm² par tex.

10. Feuille élastomère absorbante non tissée suivant une quelconque des revendications précédentes, dans laquelle ladite fibre discontinue de gonflant est de type acrylique ou polyester.

11. Feuille élastomère absorbante non tissée suivant une quelconque des revendications précédentes, dans laquelle ladite feuille a une douceur inférieure à 80 g.

12. Feuille élastomère absorbante non tissée suivant une quelconque des revendications précédentes, dans laquelle ladite feuille a une valeur de capillarité d'au moins 0,5 cm/30s.

13. Feuille élastomère absorbante non tissée suivant une quelconque des revendications précédentes, dans laquelle ladite feuille a une absorbance d'au moins 1000%.

14. Feuille élastomère absorbante non tissée suivant une quelconque des revendications précédentes, dans laquelle ladite feuille a une absorbance d'au moins 11,5 g par gramme de poids de base.

15. Feuille élastomère absorbante non tissée suivant une quelconque des revendications précédentes, dans laquelle ladite feuille a une rétention de liquide absorbé d'au moins 50%.

16. Pansement élastomère absorbant pour blessure, comprenant (1) une couche de contact de blessure perméable au fluide, souple et de faible adhérence, (2) un élément d'absorption de fluide intermédiaire conformable, l'élément comprenant la feuille élastomère absorbante non tissée suivant une quelconque des revendications précédentes,et (3) une couche de couverture douce et souple.

17. Pansement pour blessure, suivant la revendication 16, dans lequel ladite couche de contact de blessure est une feuille poreuse de fibres non tissées de petit diamètre.

18. Pansement pour blessure, suivant une quelconque des revendications 16 et 17, dans lequel ladite couche de couverture est absorbante, perméable aux liquides et perméable à la vapeur d'eau.

19. Pansement pour blessure suivant la revendication 18, dans lequel une face de ladite couche de couverture comprend une couche adhésive sensible à la pression pour venir en contact avec ledit élément d'absorption de fluide.
